# EUROPEAN PATENT SPECIFICATION

(11) **EP 0 730 651 B1**
(45) Date of publication and mention of the grant of the patent: **06.07.2005**
(21) Application number: 94929876.4
(22) Date of filing: 23.09.1994
(51) Int. Cl.: C12N 15/18, C12N 15/19, C12N 15/24, C12N 15/25, A61K 38/18, A61K 38/19, A61K 38/20

(54) **SURFACE LOOP STRUCTURAL ANALOGUES OF FIBROBLAST GROWTH FACTORS**
STRUKTURELLE ANALOGE OBERFLÄCHENSCHLAUFEN DER FIBROBLASTEN-WACHSTUMSFAKTOREN
ANALOGUES STRUCTURELS A BOUCLE DE SURFACE DE FACTEURS DE CROISSANCE DU FIBROBLAST

(30) Priority: 24.09.1993 US 126973; 15.08.1994 US 290373
(43) Date of publication of application: 11.09.1996
(73) Proprietor: Wyeth Holdings Corporation, Madison, NJ 07940 (US); YEDA RESEARCH AND DEVELOPMENT COMPANY, LTD., Rehovot 76100 (IL)
(72) Inventor: SEDDON, Andrew Peter, Monroe, NY (US); LI, Lu-Yuan, New City, NY 10956 (US); BÖHLEN, Peter, Peekskill, NY (US); EISINGER, Magdalena, Demarest, NJ (US); YAYON, Avner, 76100 Rehovot (IL)
(74) Representative: Harding, Charles Thomas
(86) International application number: PCT/US1994/010800
(87) International publication number: WO 1995/008630

(56) References cited:
- EP-A- 0 281 822
- A P SEDDON ET AL.: "Engineering of fibroblast growth factor: alteration of receptor binding specificity" BIOCHEMISTRY, vol. 34, no. 3, 1995, EASTON, PA US, pages 731-736, XP002042811
- EMBO JOURNAL, Volume 5, No. 10, issued 1986, J.A. ABRAHAM et al., "Human Basic Fibroblast Growth Factor: Nucleotide Sequence and Genomic Organization", pages 2523-2528.
- EUROPEAN JOURNAL OF BIOCHEMISTRY, Volume 188, issued 1990, M. SENO et al., "Carboxyl-Terminal Structure of Basic Fibroblast Growth Factor Significantly Contributes to its Affinity for Heparin", pages 239-245.
- BIOCHEMICAL AND BIOPHYSICAL RESEARCH COMMUNICATIONS, Volume 185, No. 3, issued 30 June 1992, M. PRESTA et al., "Structure-Function Relationship of Basic Fibroblast Growth Factor: Site-Directed Mutagenesis of a Putative Heparin-Binding and Receptor-Binding Region", pages 1098-1107.
- BIOCHEMISTRY, Volume 30, issued 1991, W.F. HEATH et al., "Mutations in the Heparin-Binding Domains of Human Basic Fibroblast Growth factor Alter its Biological Activity", pages 5608-5615.
- DATABASE BIOSIS [Online] 1989 HYNES: 'transfer of a beta-turn structure to a new protein context' Database accession no. 3424649
- ERIKSSON A.E. ET AL: 'Three-dimensional structure of human basic fibroblast growth factor' PROC. NATL. ACAD. SCI. USA vol. 88, April 1991, pages 3441 - 3445, XP002936511

## Description

### Technical Field of the Invention

This invention relates to fibroblast growth factor analogues having different primary structures in a surface loop that connects the ninth and tenth β-strands. The analogues retain overall secondary and tertiary protein structural similarities to the original factors but exhibit different biological properties and receptor binding specificity profiles.

### Background of the Invention

Polypeptide growth factors are hormone-like modulators of cell proliferation and differentiation. Growth factors are responsible for the regulation of a variety of physiological processes, including development, regeneration, and wound repair, and have been associated with normal as well as with pathophysiological processes. Numerous growth factors have been identified in various tissues and cells, and names that have been applied to these factors include epidermal growth factor, platelet-derived growth factor, nerve growth factor, hematopoietic growth factors, and fibroblast growth factor.

Fibroblast growth factor (FGF) was first described as an activity derived from bovine brain or pituitary tissue which was mitogenic for fibroblasts and endothelial cells. It was later noted that the primary mitogen from brain was different from that isolated from pituitary. These two factors were named acidic and basic FGF, respectively, because they had similar biological activities but differed in their isoelectric points. Acidic and basic FGF are proteins containing approximately 154 amino acids. Their amino acid sequences are related, with approximately 55% sequence identity between them.

Acidic and basic fibroblast growth factors are now known to be members of a larger family of heparin-binding growth factors that collectively trigger a variety of biological responses in many cell types, including those of mesoderm and neuroectoderm origin, such as endothelial cells, smooth muscle cells, adrenal cortex cells, prostatic and retina epithelial cells, oligodendrocytes, astrocytes, chrondocytes, myoblasts, and osteoblasts. As original family members, acidic and basic FGF are now denoted FGF-1 and FGF-2, respectively. Seven other members of the family have been identified on the basis of their modulation of cell proliferation and differentiation, and their sequence homology to other FGFs.

In addition to eliciting a mitogenic response that stimulates cell growth, fibroblast growth factors can stimulate a large number of cell types to respond in a non-mitogenic manner. These activities include promotion of cell migration into wound areas (chemotaxis), initiation of new blood vessel formulation (angiogenesis), modulation of nerve regeneration and survival (neurotrophism), modulation of endocrine functions, and stimulation or suppression of specific cellular protein expression, extracellular matrix production and cell survival (Baird, A., and Böhlen, P., *Handbook of Exp. Pharmacol. 95*(1): 369-418, Springer, 1990). These properties provide a basis for using fibroblast growth factors in therapeutic approaches to accelerate wound healing, nerve repair, collateral blood vessel formation, and the like. For example, fibroblast growth factors have been suggested to minimize myocardium damage in heart disease and surgery (U.S. Pat. No. 4,378,347 to Franco).

Current research regarding FGF-2 and other FGFs has centered on the molecular details of the receptor-mediated pathways by which their diverse physiological activities are expressed, as a way to gain information for the design of therapeutically useful agents that can either mimic or inhibit the action of these factors. Since the primary structure of FGF-2 isolated from a variety of sources is known, and bovine and human FGF-2 have been cloned and expressed in *E. coli* and *S. cervisiae,* recent attention has focused on secondary and tertiary structure.

The 3-dimensional structures of FGF-1 and FGF-2 have been determined (Eriksson, E.A., *et al., Proc. Nat. Acad. Sci. USA 88*: 3441-3445 (1991), Zhang, *J., et al*., *Proc. Nat. Acad. Sci. USA 88*: 3446-3450 (1991), and Zhu, H., *et al*., *Science 251:* 90-93 (1991)). In these studies, FGF-1 and FGF-2 were shown to exhibit a folding pattern strikingly similar to that observed for the cytokine interleukin-1α, and interleukin-1β (IL-1α and IL-1β), protein factors produced by macrophages and T-cells in response to antigenic or mitogenic stimulation, though the primary structures of interleukin-1 polypeptides have only about a 10% amino acid sequence correspondence to FGFs.

The overall structure of FGF-2 can be described as a trigonal pyramid where each of the three sides are built of two β-strands together forming a β-sheet barrel of six antiparallel strands (Eriksson, E.A., *et al., Proc. Nat. Acad. Sci. USA 88:* 3441-3445 (1991)). The base of the pyramid is built of six additional β-strands extending from the three sides of the pyramid to close one end of the barrel for a total of twelve β-strands. Thus, a threefold repeat is observed in the folding of the polypeptide chain and a pseudo-three-fold axis passes through the center of the base of the molecule and extends through the apex of the pyramid (*ibid*.). Of the amino acids conserved within the FGF family of proteins, most are located within the core β-strand regions of FGF-2, supporting the expectation that each of these proteins has an overall 3-dimensional structure similar to that of FGF-2.

The biological responses of FGF are mediated by the heparan sulfate-dependent binding of the growth factor to specific cell surface receptors (Givol, D., and Yayon, A., *FASEB J. 6*: 3362-3369 (1992) and Jaye, M., *et al*., *Biochim. Biophys. Acta 1135:* 185-199 (1992)), yet the molecular interactions of heparin and receptor with FGF and the exact nature of the events of the signal transduction pathway are unknown. Studies employing synthetic peptides related to the FGF sequence showed that FGF-2 (33-77) and (106-129) bind to heparin and act as weak partial agonists and antagonists in a mitogenic assay of FGF activity (Baird, A., *et al. Proc. Nat. Acad. Sci. USA 85:* 2324-2328 (1988)). The same study identified a sequence, FGF-2 (115-124), involved in receptor binding. The segment begins in the middle of the ninth β-strand, makes a somewhat open loop on the surface of the folded molecule, and terminates at the beginning of the tenth β-strand. This sequence (118-122) in the native protein forms a small surface loop that is close to a cluster of basic surface residues that may form a putative heparin binding site (Zhang, J., *et al., Proc. Nat. Acad. Sci. USA 88*: 3446-3450 (1991)).

This sequence also contains Thr-121, which can be phosphorylated by a CAMP-dependent protein kinase (Feige, J.J., and Baird, A., *Proc. Nat. Acad. Sci. USA 86:* 3174-3178 (1989)); Thr-121 is denoted in the paper as Thr-112 since the investigators employed the N-terminally truncated form of the polypeptide, which exhibits full biologic activity but has 146 rather than the usual 154 amino acids). Phosphorylation of Thr-121 results in the generation of a form of the protein that exhibits an increased capacity to compete with radiolabelled FGF-2 binding to its receptor, but no difference in the biological properties of the phosphorylated and nonphosphorylated forms of the protein were observed (*ibid*.). In contrast to FGF-2, FGF-1 is not a substrate for the kinase. The data are consistent with the hypothesis that the sequence 115-124 is involved in receptor binding, but they do not define the complete receptor binding domain of the molecule, nor do they demonstrate the physiological significance of phosphorylation of Thr-121.

In addition to heparin and receptor binding regions, there is evidence that specific sequences in FGF influence ligand-induced signal transduction. For example, deletion of residues 27-32 of FGF-2 (Lys-Asp-Pro-Arg-Leu) or mutation of the basic residues Arg-118, Lys-119, Lys-128, and Arg-129 did not appear to affect the mitogenic activity of the protein, but eliminated activation of plasminogen activator gene expression (Eur. Pat. Ap. Pub. No. 363,675 to Bergonzoni, L., *et al*., and Presta, M., *et al., Biochem. Biophys. Res. Com. 185*: 1098-1107 (1992)).

At least four different fibroblast growth factor receptors (FGFR) have been identified (Werner, S., *et al., Mol. Cell. Bio. 12*: 82-88 (1992)), and functional differences between different receptor forms have been observed. Different FGF receptor forms derived from the same gene via alternative splicing have different ligand binding properties, and analogous splice variants from different FGF receptor genes bind different members of the FGF family (Johnson, D.E., and Williams, L.T., *Adv. Can. Res. 60:* 1-41 (1993)). Thus, fibroblast growth factor receptors exhibit a multitude of structural variants, and considerable cross-reactivity between receptors and their various ligands (Yayon, A., et *al., EMBO J. 11*: 1885-1890 (1992)). Though the carboxy-terminal region of the third immunoglobulin-like domain appears to be a structural element that defines specificity of different FGF members (Werner, *et al*., and Yayon, *et al*., cited above), the precise nature of FGF-receptor-heparin interactions and the protein residues involved have yet to be elucidated.

### Summary of the Invention

The invention provides new structural analogues of fibroblast growth factor-2.

Such fibroblast growth factor-2 analogues have binding and biological activities that are pharmacologically dissociated, *i.e*., that bind to an FGF receptor, which may be the same or different from a receptor that normally binds the wild-type factor, and/or exhibits different biological properties from that observed in the wild-type factor.

The invention thus provides fibroblast growth factor structural analogues such as structural analogues of human fibroblast growth factor-2, which have all or part of a surface loop replaced with another amino acid sequence. Such analogues are set out in Claim 1.

These fibroblast growth factor structural analogues have amino acid changes in surface loops, or sequences adjacent to the surface loops, such that the overall secondary and tertiary structures of the polypeptides are not significantly perturbed, and the analogues bind to heparin. More particularly, this invention provides fibroblast growth factor-2, structural analogues having an amino acid sequence replacement in the ninth or tenth β-strand of the factor, or the sequence that corresponds to the surface loop that connects the ninth and tenth β-strands, such that the interaction of β-strands nine and ten with adjacent strands is not significantly perturbed, and the analogue binds to heparin. The analogues exhibit binding affinity to a fibroblast growth factor receptor. Binding specificity of the analogues is preferably different from the binding specificity of the native factor to the corresponding receptor. In one embodiment, the analogue stimulates *in vivo* angiogenesis.

The fibroblast growth factor structural analogues have an amino acid sequence substitution in the surface loop that extends from the ninth to the tenth β-strand. All or part of the amino acid sequence in the growth factor surface loop extending from the ninth to the tenth β-strands is replaced by another amino acid sequence such as a corresponding amino acid sequence derived from fibroblast growth factor-1.

We therefore specifically provide, according to the invention an FGF-2 analogue in which amino acids corresponding to FGF-2 amino acids 118 to 122 of SEQ ID NO 1 have been replaced with an amino acid sequence selected from the group consisting of:
(a) FGF-1 amino acids 115-121 of SEQ ID NO 2;
(b) FGF-1 amino acids 115-121 of SEQ ID NO 2; except that His121 is replaced by Asn121;
(c) FGF-3 amino acids 132-152 of SEQ ID NO 4;
(d) FGF-4 amino acids 172-176 of SEQ ID NO 5;
(e) FGF-5 amino acids 176-186 of SEQ ID NO 6;
(f) FGF-6 amino acids 174-178 of SEQ ID NO 7;
(g) FGF-7 amino acids 154-162 of SEQ ID NO 8;
(h) FGF-8 amino acids 144-148 of SEQ ID NO 9;
(i) FGF-9 amino acids 151-161 of SEQ ID NO 10; and
(j) interleukin-1β amino acids 231-235 of SEQ ID NO 3,
wherein said analogue is capable of binding to heparin. Analogues having loop flanking sequences changed, and peptide fragment analogues containing the loop sequences are correspondingly provided by the invention.

For example, human fibroblast growth factor-2 structural analogues having amino acid sequence substitutions in residues 118 to 122 result in factors that retain the same overall secondary and tertiary structure as FGF-2, but exhibit different biological properties. In these exemplary embodiments, an FGF-2 loop sequence is replaced with any insert set out in (a) to (j) above. Some derivatives are prepared by changing residues 115 to 124; others are prepared by changing loop residues 118 to 122. In one embodiment, for example, the FGF-2 loop is replaced with corresponding amino acids derived from FGF-1 from the same or another species; in another, an interleukin-1β from the same or another species is used; and, in another, a loop from FGF-7 from the same or another species is used. As summarized above, these analogues bind heparin and a native fibroblast growth factor receptor.

The invention also provides DNA encoding the fibroblast growth factor derivatives, biologically functional circular plasmid or viral DNA vectors comprising the DNA, and procaryotic or eucaryotic host cells such as *E. coli* transformed or transfected with the vectors in a manner allowing the host cell to express the new factors.

### Brief Description of the Figures

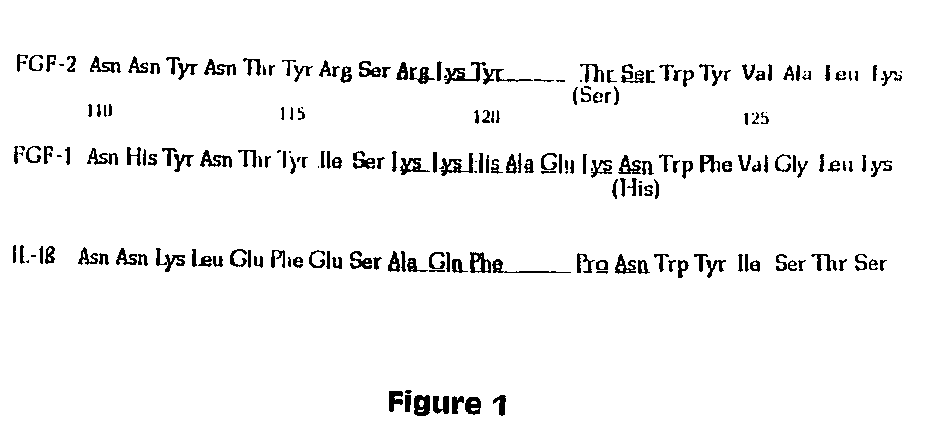

Figure 1 sets out sequence alignment of human FGF-2, FGF-1 and IL-1β in the loop region (identified in the Sequence Listing section hereinafter as ID NOs 1 to 3). The sequences underlined correspond to the surface loop located at the end of the ninth β-strand. The residues in parenthesis indicate the residues found in the bovine sequences.

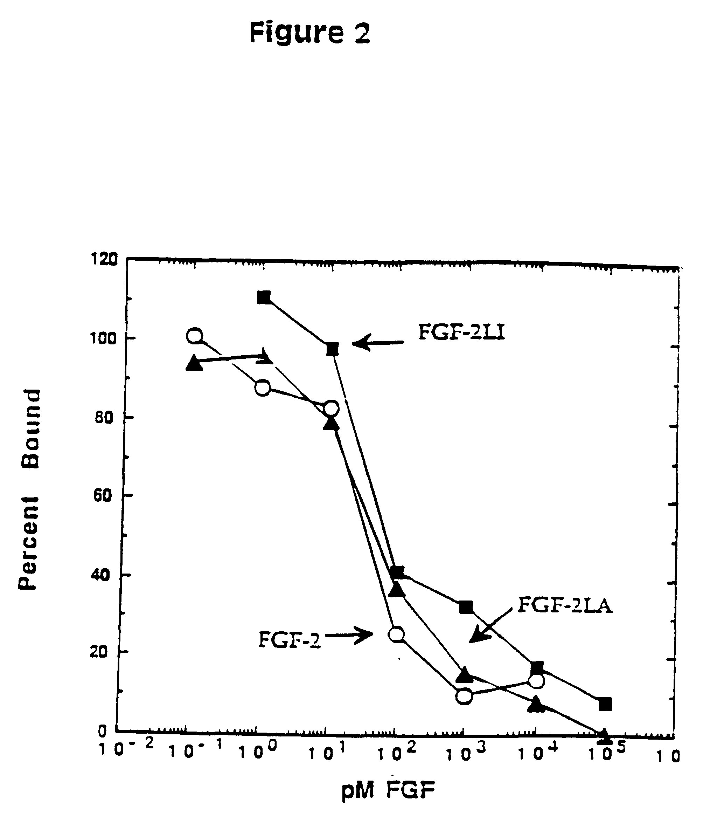

Figure 2 plots competitive receptor binding of confluent baby hamster kidney cells incubated with ¹²⁵I-FGF-2 and indicated concentrations of competitors FGF-2 (open circles), structural analogue FGF-2LI (described in greater detail hereinafter, closed squares), and structural analogue FGF-2LA (described hereinafter, closed triangles).

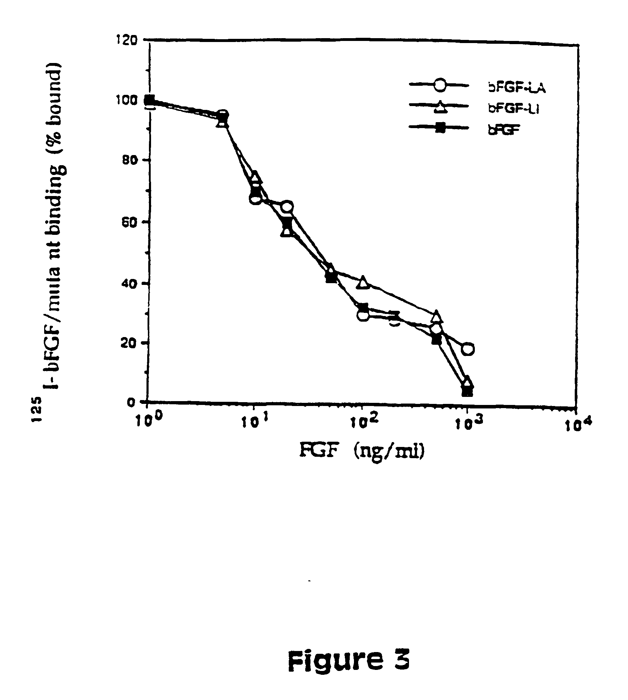

Figure 3 plots the binding of radioiodinated FGF-2 (closed squares), FGF-2LA (open circles), and FGF-2LI (open triangles) to FGF-receptor type 1 present on NIH 3T3 cells as a function of added unlabelled FGF proteins.

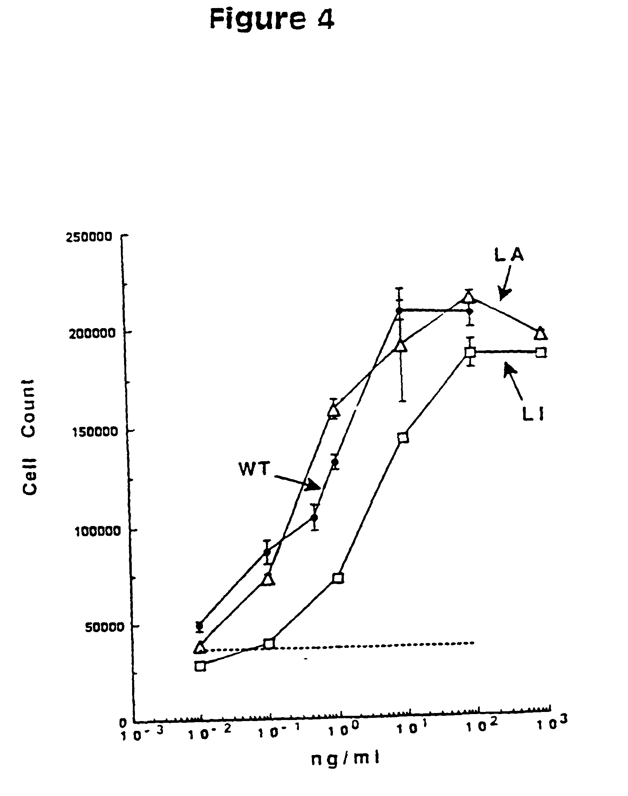

Figure 4 shows endothelial cell growth of adult bovine aortic arch endothelial cells plated at 8000 cells/well and incubated for 5 days with the indicated concentrations of FGF-2 (closed circles), FGF-2LI (open squares), and FGF-2LA (open triangles).

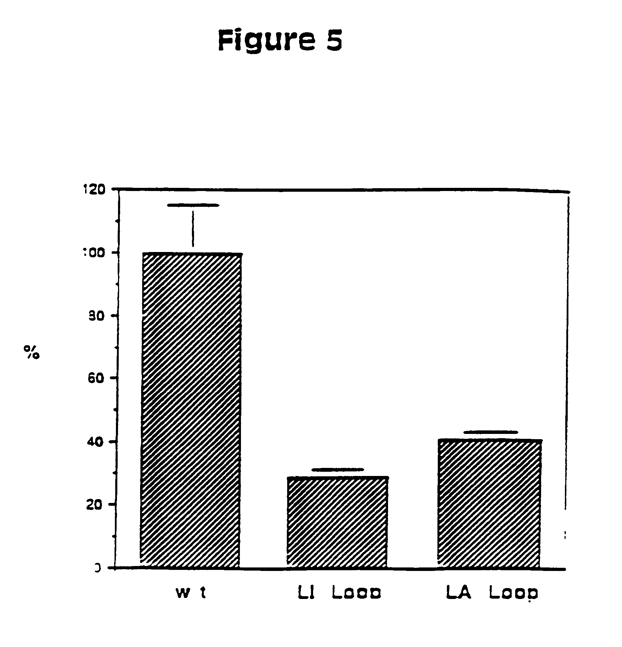

Figure 5 is a bar graph depicting production of cell-associated urokinase-type plasminogen activator induced by wild-type or mutant FGF-2 (10 ng/ml).

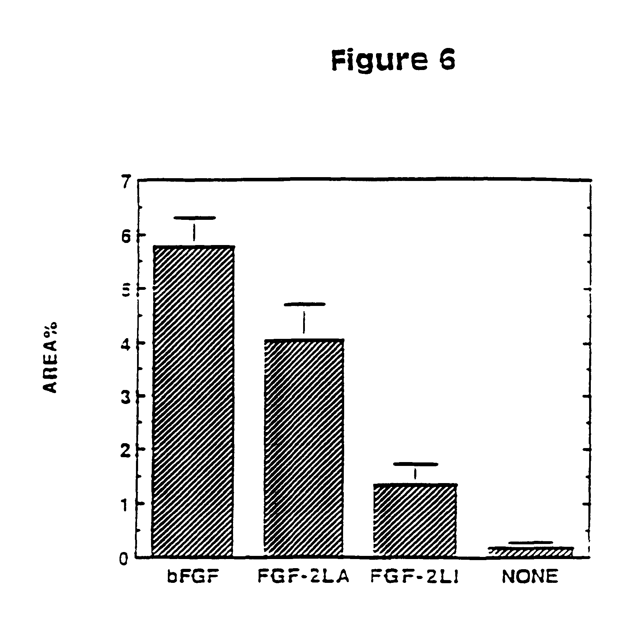

Figure 6 is a bar graph illustrating the potency of *in vitro* angiogenic activities of wild-type or mutant FGF-2 (10 ng/ml) as measured by their abilities to induce formation of tube-like structures by ABAE cells cultured on a type I collagen gel. The data are from a typical experiment in which cell cultures of identical conditions are maintained in duplicate wells. Three to four areas of each well are examined by image analysis and the mean value and standard deviation are presented.

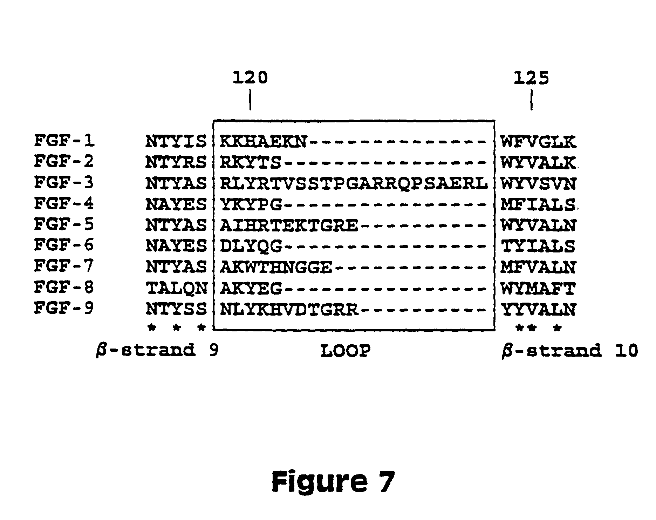

Figure 7 presents an amino acid sequence comparison among the nine members of the FGF family between residues corresponding to amino acid residues 113 and 128 in FGF-2 (SEQ ID NO 1; FGF-1 is set out as SEQ ID NO 2, and the other FGFs are sequentially set out as SEQ ID NOs 4 to 10, except that *His*121 of the FGF-1 bovine sequence is replaced by Asn in the human sequence). The numbering system is relative to FGF-2 and the asterisks refer to identical and conserved residues. Residues located between the FGF-2 locations Ser117 to Trp123 mark the positions where other FGFs contain inserts and changes. To illustrate homologous sequences in the ninth and tenth β-strands and in the loop region, the figure employs standard one-letter nomenclature for the amino acids: A, Ala; C, Cys; D, Asp; E, Glu; F, Phe; G, Gly; H, His; I, Ile; K, Lys; L, Leu; M, Met; N, Asn; P, Pro; Q, Gln; R, Arg; S, Ser; T, Thr; V, Val; W, Trp; and Y, Tyr.

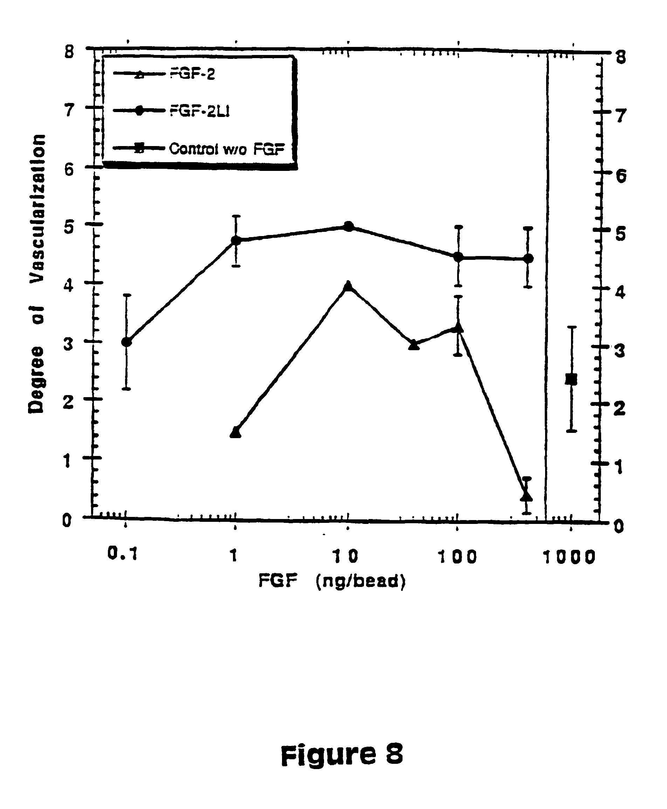

Figure 8 shows a comparison of the degree of neovascularization induced in a rabbit ear chamber model by FGF-2 (closed triangles) and FGF-2LA (closed circles). A control without FGF is represented by closed squares.

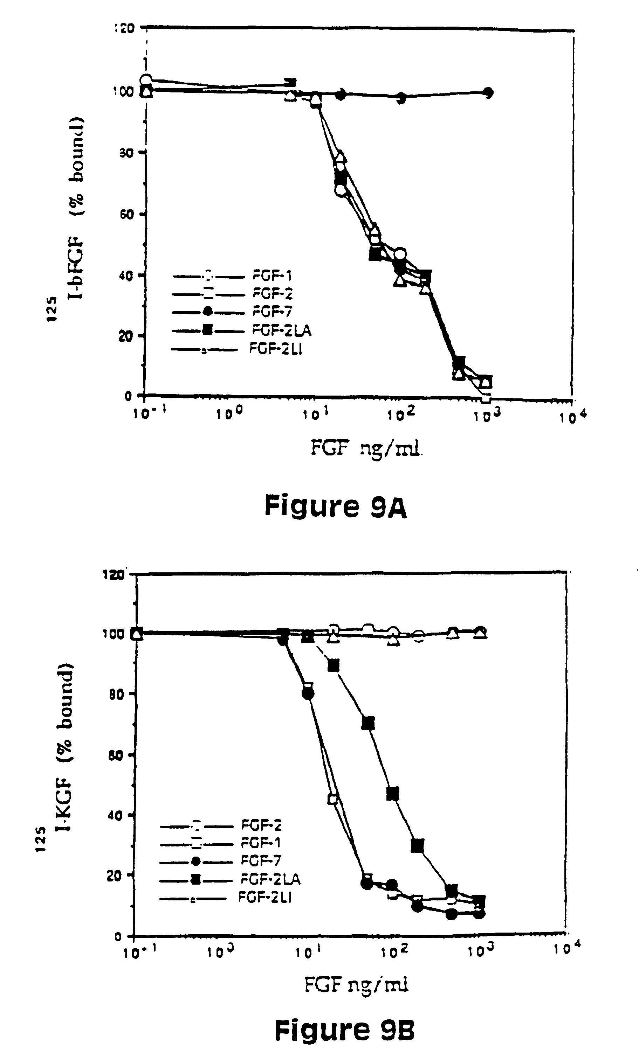

Figure 9A plots the binding of ¹²⁵I-FGF-2 as a function of added FGF-1 (open circles), FGF-2 (open squares), FGF-7 (closed circles), FGF-2LA (closed squares), and FGF-2LI (open triangles) to soluble recombinant FGF-receptor type 1 as a function of added unlabelled FGF proteins. Figure 9B plots a similar binding profile, except that soluble recombinant FGF-2(IIIc) is employed.

### Detailed Description of the Invention

This invention is based upon the finding that changes in fibroblast growth factor surface loop residues in a manner that does not perturb the overall secondary and tertiary structure of the FGF molecule yields an array of structural analogues having varied and desirable physiological properties, including FGF agonists and potential antagonists. Changes in loop sequences, or sequences adjacent to loop sequences, yield analogues that bind to heparin and exhibit binding affinity to a native fibroblast growth factor receptor.

By "antagonist" is meant any substance that tends to nullify the action of another, as one, for example, that binds to a cell receptor without eliciting a biological response. For FGFs, antagonists include, but are not limited to, any substance that binds to an FGF receptor but does not stimulate proliferation or migration of fibroblasts, endothelial cells and astroglial cells. In contrast, by "agonist" is meant any substance that has affinity for and stimulates physiological activity at cell receptors normally stimulated by naturally occurring factors, including mitogenic activity, angiogensis, chemotaxis, and the like. As used herein, the terms are not limiting, and a single factor may exhibit antagonism to one biological function mediated by native factor, and agonism to another function. Where this occurs, the biological activities of native factor are said to be pharmacologically dissociated in the analogue.

A protein is defined herein as a fibroblast growth factor (FGF) if it shows significant sequence and three-dimensional structural homology to other members of the FGF family, FGF-like activity in *in vitro* or *in vivo* assays and binds to heparin or heparin-like substances. By "heparin" is meant the heterogeneous, sulfated anionic polysaccharide composed of D-glucuronic acid and D-glucosamine, bound to a protein core as the "proteoglycan" or in a free form as the "aglycan", that may or may not have anticoagulant properties. "Heparin- like" substances are molecules that have oligosaccharide structures related to the heparins, but may or may not have anticoagulant activity. Any type of fibroblast growth factor or mutein or derivative is encompassed by this invention, particularly human fibroblast growth factor, as well as loop sequence peptides that exhibit antagonist or agonist properties, or both.

Broadly speaking, the fibroblast growth factor analogues of this invention include structural analogues of fibroblast growth factor having at least one amino acid deletion, insertion or replacement in a β-strand, or a loop connecting two β-strands such that adjacent strands are not perturbed, and the molecule retains its overall secondary and tertiary structure. Secondary and tertiary structure is determined by heparin binding, binding to an FGF receptor, spectroscopy (using ultraviolet, visible, or circular dichroic light), X-ray crystallography, and biological activities.

Particularly preferred are FGF sequence replacements in a loop connecting two β-strands, analogous to the structural cassette or module replacements of turns with retention of parent conformation disclosed for enzymes by Hynes, T.R., *et al., Nature 339*: 73-76 (1989). In some embodiments, the ninth or tenth β-strand of the factor, or the sequence that corresponds to the surface loop that connects the ninth and tenth β-strands, are replaced with any size or composition amino acid sequence insert such that overall molecular folding is not significantly perturbed. In certain embodiments, the surface loop is replaced with a surface loop from another factor. Thus, this invention specifically encompasses loop and loop stem and anchor replacements with structurally related and unrelated sequences, including random loops, longer or shorter loops, and differently charged loops and their stems and anchors. It also encompasses peptide fragments that mimic these loops in binding studies and biological assays.

As set out more fully below, numbering of the amino acids in these strands is, for convenience, made with reference to FGF-2. Perturbation and strand adjacency are herein defined in a three-dimensional sense and measured as described above. Overall folding is maintained in the analogues. Preferred analogues bind to an FGF receptor with an affinity that is greater than, or up to 100-times lower than the binding of a corresponding native FGF family member exhibiting affinity to the receptor.

Receptors that many of the analogues typically bind include native baby hamster kidney (BHK) cell fibroblast growth factor receptor, NIH 3T3 cell receptors, or other receptors specific to particular FGFs. Binding preferably exhibits an affinity substantially similar or superior to the binding of the corresponding native factor to that receptor. An advantage of the invention is that the loop manipulation results in analogues exhibiting similar binding profiles to the corresponding factors from which the loops are derived. Thus, certain analogues of the invention target receptors that the native factor does not bind, including receptors on different cell types. For example, factors that ordinarily bind to endothelial cell receptors can be engineered to bind to epithelial cell receptors, and so forth. Examples are given hereinafter. One embodiment stimulates angiogenic activity *in vivo*.

Especially preferred FGF-2 structural analogues are those in which the factor has a substitution as set out above in the sequence of the surface loop that extends from the ninth to the tenth β-strand and protrudes from the surface when the molecule is folded as described by Eriksson, *et al*., Zhu, *et al*., and Zhang, *et al*., cited above, and has the overall secondary and tertiary structure of the native factor.

For example, structural FGF-2 analogues of the invention include analogues having an amino acid sequence substitution in surface loops that include fibroblast growth factor-1 sequence Lys115-Lys116-His117-Ala118-G1u119-Lys120-Asn121 (amino acids 115-121 of SEQ ID NO 2); fibroblast growth factor-3 sequence Arg-Leu-Tyr-Arg-Thr-Val-Ser-Ser-Thr-Pro-Gly-Ala-Arg-Arg-Gln-Pro-Ser-Ala-Glu-Arg-Leu (amino acids 132-152 of SEQ ID NO 4), fibroblast growth factor-4 sequence Tyr-Lys-Tyr-Pro-Gly (amino acids 172-176 of SEQ ID NO 5), fibroblast growth factor-5 sequence Ala-Ile-His-Arg-Thr-Glu-Lys-Thr-Gly-Arg-Glu (amino acids 176-186 of SEQ ID NO 6), fibroblast growth factor-6 sequence Asp-Leu-Tyr-Gln-Gly (amino acids 174-178 of SEQ ID NO 7),
fibroblast growth factor-7 (also known as keratinocyte growth factor or KGF) sequence Ala-Lys-Trp-Thr-His-Asn-Gly-Gly-Glu (amino acids 154-162 of SEQ ID NO 8), fibroblast growth factor-8 sequence Ala-Lys-Tyr-Glu-Gly (amino acids 144-148 of SEQ ID NO 9), fibroblast growth factor-9 sequence Asn-Leu-Tyr-Lys-His-Val-Asp-Thr-Gly-Arg-Arg (amino acids 151-161 of SEQ ID NO 10) and interleukin-1β amino acids 231-235 of SEQ ID NO 3. A comparison of surface loop sequences in the FGF family is depicted in Figure 7.

Changes in neighboring regions that affect the loop, including loop stems and anchor points, are encompassed by the invention, so long as the overall folding of the molecule is not perturbed. Thus, analogues of the invention include structures having mutations, particularly sequence replacements, in fibroblast growth factor-2 sequence Arg118-Lys119-Tyr120-Thr121-Ser122 (amino acids 118-122 of SEQ ID NO 1).

By "FGF-2" is meant any fibroblast growth factor-2 exhibiting biologic activity including the 146-amino acid polypeptide originally isolated and sequenced, the 154 amino acid form currently thought to be the full polypeptide, truncated forms exhibiting activity, extended forms such as placental FGF, higher molecular weight N-terminally extended forms described in the literature and analogues including derivatives and muteins of any of these. The term specifically includes natural FGF-2 extracted from mammalian tissue as well as recombinant polypeptides expressed from cloned DNA in *E. coli* or *S. cerevisiae* from any species or expressed in insect or mammalian cells with appropriate vectors.

Human FGF-2 is preferred in many embodiments. Human FGF-2 having 9th or 10th β-strands which can be manipulated according to the invention includes, but is not limited to, FGF-2 having amino acid additions, amino acid substitutions, and amino acid deletions, including deletions of portions of the amino or carboxyl terminus, and chimeric proteins containing FGF at the N-or C-terminus of another protein. Example FGF-2s which can be manipulated according to the invention include those having cysteine substituted with a neutral amino acid such as serine, or aspartic acid, arginine, glycine, serine, or valine substituted with other acids suggested to have enhanced stability in Eur. Pat. Ap. Pub. No. 281,822 to Seno, *et al*.; muteins formed by replacing at least one, and more preferably two, of the cysteines found in natural FGF-2 with a different amino acid residue to yield a more stable analogue (Eur. Pat. Ap. Pub. No. 320,148 to Arakawa and Fox); muteins lacking amino acids from the carboxyl terminus and, optionally, having amino acid replacements suggested to have improved stability while retaining activity in Eur. Pat. Ap. Pub. No. 326,907 to Seno, *et al*.; mutants lacking a substantial part of the amino-or carboxyl-terminus such as those described by Seno, *et al.; Eur. J. Biochem*. 188: 239-245 (1990); muteins having various point mutations or an N-terminal deletion suggested in Eur. Pat. Ap. Pub. No. 298,723 to Fiddes, *et al*.; the M1-bFGF to M6-bFGF muteins containing missing and substituted amino acids disclosed in Eur. Pat. Ap. Pub. No. 363,675 to Bergonzoni, cited above; readily expressed FGF prepared by replacement of Ala-3 and Ser-5 of recombinant FGF with Glu as described in Seddon, A.P. *et al., Annals N.Y. Acad. Sci. 638*: 98-108 (1991) and analogues thereof; and the like.

In the practice of this invention, a fibroblast growth factor derivative of this invention such as a human FGF-2 structural analogue is prepared by substituting the amino acids in the sequence between residues 118 (Arg) and 122 (Ser). As defined above, the numbering convention for other FGF analogues is relative to FGF-2.

The fibroblast growth factor structural analogues have an amino acid sequence replacement in the ninth or tenth β-strand of the factor, or the sequence that corresponds to the surface loop that connects the ninth and tenth β-strands. Amino acid sequences are herein defined as a sequence of at least three amino acids, typically at least five amino acids. Exemplary amino acid sequences are set out above.

A structural analogue of this invention exhibiting desirable biological properties more particularly described below comprises a fibroblast growth factor-2 derivative having surface loop amino acids Arg118-Lys119-Tyr120-Thr121-Ser122 (amino acids 118-122 of SEQ ID NO 1) replaced with corresponding amino acid sequence Ala115-Gln116-Phe117-Pro118-Asn119 (amino acids 231-235 of SEQ ID NO 3) from human interleukin-1β, denoted FGF-2LI in the Examples that follow (SEQ ID NO 15). In another embodiment, the analogue comprises a human fibroblast growth factor-2 derivative having surface loop amino acids Arg118-Lys119-Tyr120-Thr121-Ser122 (amino acids 118-122 of SEQ ID NO 1) replaced with corresponding amino acid sequence Lys115-Lys116-His117-Ala118-Glu119-Lys120-His121 (amino acids 115-121 of SEQ ID NO 2) derived from bovine FGF-1, denoted FGF-2LA in the Examples that follow (SEQ ID NO 16). In yet another embodiment, the analogue comprises a human fibroblast growth factor-2 derivative having surface loop amino acids 118 to 122 replaced with a corresponding 9-residue amino acid loop sequence from FGF-7 (also known as Keratinocyte Growth Factor), Ala-Lys-Trp-Thr-His-Asn-Gly-Gly-Glu from FGF-7 (residues 154-162 of SEQ ID NO 8).

The novel fibroblast growth factor structural analogues of this invention are prepared by point mutations, sequence alterations or polypeptide assembly from constituent amino acids or peptides using chemical, biochemical or physical means known to those skilled in the art. Alternatively, the novel fibroblast growth factor analogues of this invention are prepared by recombinant protein synthesis involving preparation of DNA encoding a surface loop mutein, insertion of that DNA into a vector, expression of the vector in host cells, and isolation of the mutant FGF thereby produced.

DNA encoding the FGF structural analogues of this invention are prepared by altering a gene of fibroblast growth factor by nucleotide deletions, nucleotide additions, or point mutations produced using standard means. Illustrations are set out in Examples 1 and 2. Because of the degeneracy of the genetic code, a variety of codon change combinations can be selected to form DNA that encodes the FGF analogues of this invention, so that any nucleotide deletion(s), addition(s), or point mutation(s) that result in a DNA encoding loop mutant FGF are encompassed by this invention. Since certain codons are more efficient for polypeptide expression in certain types of organisms, the selection of fibroblast gene alterations to yield DNA material that codes for the FGF muteins of this invention are preferably those that yield the most efficient expression in the type of organism which is to serve as the host of the recombinant vector. Altered codon selection may also depend upon vector construction considerations.

Fibroblast growth factor DNA starting material which is altered to form DNA coding for the FGF analogues of the invention may be natural, recombinant or synthetic. Thus, DNA starting material is isolated from tissue or tissue culture, constructed from oligonucleotides using conventional methods, obtained commercially, or prepared by isolating RNA coding for FGF from fibroblasts, and using this RNA to synthesize single-stranded cDNA which is used as a template to synthesize the corresponding double stranded DNA.

Illustrating the present invention are cloned complementary DNA sequences defining human FGF-2 analogues such as that constructed in Examples 1 and 2. Also encompassed are DNA sequences homologous or closely related to complementary DNA described herein, namely DNA sequences which hybridize, particularly under stringent conditions that result in pairing only between nucleic acid fragments that have a high frequency of complementary base sequences, to FGF analogue cDNA, and RNA corresponding thereto. In addition to the FGF-encoding sequences, DNA encompassed by this invention may contain additional sequences, depending upon vector construction sequences, that facilitate expression of the gene.

DNA encoding the FGF analogues of this invention, or RNA corresponding thereto, are then inserted into a vector, *e.g*., a pBR, pUC, pUB or pET series plasmid, and the recombinant vector used to transform a microbial host organisms. Host organisms useful in the invention are bacterial (*e.g., E. coli or B. subtilis*), yeast (*e.g., S*. *cervisiae*)*,* mammalian (*e.g*., mouse fibroblast), or insect cells. This invention thus also provides novel, biologically functional viral and circular plasmid RNA and DNA vectors incorporating RNA and DNA sequences describing the FGF analogues generated by standard means. Culture of host organisms stably transformed or transfected with such vectors under conditions facilitative of large scale expression of the exogenous, vector-borne DNA or RNA sequences and isolation of the desired polypeptides from the growth medium, cellular lysates, or cellular membrane fractions yields the desired products. An example of expression of FGF-2 muteins in *E. coli* is given in Example 3.

The present invention provides for the total and/or partial manufacture of DNA sequences coding for FGF-2 loop mutants, and including such advantageous characteristics as incorporation of codons preferred for expression by selected non-mammalian hosts, provision of sites of cleavage by restriction by endonuclease enzymes, and provision of additional initial, terminal or intermediate DNA sequences which facilitate construction of readily expressed vectors. Correspondingly, the present invention provides for manufacture (and development by site specific mutagenesis of cDNA and genomic DNA) of DNA sequences coding for microbial expression of FGF analogues which differ from the forms specifically described herein in terms of identity or location of one or more amino acid residues (i.e., deletion analogues containing less than all of the residues specified for human FGF-2, and/or substitution analogues wherein one or more residues are added to a terminal or medial portion of the polypeptide), and which share the biological properties of FGF-2 analogues described herein.

DNA (and RNA) sequences of this invention code for all sequences useful in securing expression in procaryotic or eucaryotic host cells of polypeptide products having at least a part of the primary structural conformation, and one or more of the biological properties of FGF analogues which are comprehended by: (a) the DNA sequences encoding FGF-2 loop muteins as described herein, or complementary strands; (b) DNA sequences which hybridize to DNA sequences defined in (a) or fragments thereof; and (c) DNA sequences which, but for the degeneracy of the genetic code, would hybridize to the DNA sequences defined in (a) and (b) above. Specifically comprehended are genomic DNA sequences encoding allelic variant forms of FGF analogues included therein, and sequences encoding loop mutein RNA, fragments thereof, and analogues wherein RNA or DNA sequences may incorporate codons facilitating transcription or RNA replication of messenger RNA in non-vertebrate hosts.

Isolation and purification of microbially expressed polypeptides provided by the invention are by conventional means including, for example, preparative chromatographic separations such as that illustrated Example 3, and immunological separations, including monoclonal and/or polyclonal antibody preparations.

As summarized above and described in detail in the Examples below, two example FGF-2 structural analogues of this invention are FGF-2LA (SEQ ID NO 16) and FGF-2LI (SEQ ID NO 15), FGF-2 sequences having loop residues 118 to 122 replaced by corresponding sequences from bovine FGF-1 and human interleukin-1β, respectively, expressed in *E. coli*. These mutations have no apparent effect on either heparin binding, estimated by the concentration of NaCl required to elute the protein from heparin Sepharose, or on the ability of the proteins to compete with I¹²⁵-FGF-2 for binding to the FGF receptors present on baby hamster kidney cells and NIH 3T3 cells (Example 4). The loop analogues are mitogenically active in bovine endothelial cell proliferation assays. Both analogues exhibit significantly reduced capability to induce urokinase-type plasminogen activator. In an *in vitro* angiogenesis assay, FGF-2LA exhibits capillary-like tube formation comparable to wild-type FGF-2. In the same *in vitro* assay, FGF-2LI exhibits much less induction of capillary-like structures, but it significantly stimulates angiogenesis in an *in vivo* assay.

Another FGF-2 loop mutant, FGF-2LK (described in greater detail in the Examples), contains a 9-amino acid sequence from FGF-7 (Keratinocyte Growth Factor). FGF-2 does not bind to FGF-7 receptor (FGFR2 IIIb) on keratinocytes, and FGF-7 does not bind to FGF receptor type 1 (FGFR1). Replacement of the loop sequence in FGF-2 with that from FGF-7 results in an apparent decrease in the affinity of the protein to heparin. In receptor binding experiments, the FGF-2LK protein is unable to displace or compete with binding of FGF-2 to FGF receptor type 1, but competes with FGF-7 to receptor type 2 (IIIb) whereas FGF-2 does not compete with binding. Thus, the loop sequence confers receptor-ligand specificity and allows for the binding of FGF-2LK mutant to a receptor subtype (FGFR IIIb) that binds FGF-7 but not FGF-2. Conversely, the loop sequence from FGF-7 abolishes binding of the protein to FGF receptor 1.

Introduction of new primary structural elements in the FGF surface loop without significantly perturbing the overall secondary and tertiary structure of the molecule provides an array of FGF structural analogues that give rise to altered properties from one FGF type to another, and thus a means to modulate the activities of FGF proteins, a means to pharmacologically dissociate the biological activities of the proteins, and a means to introduce new activities. As illustrated in the Examples that follow, FGF loop analogues can be structured to bind to the same receptors as corresponding native FGF, or to different receptors, particularly to receptors corresponding to the loop rather than to the native FGF.

FGF antagonists exhibiting reduced biological activity are useful as anticancer and antiproliferative agents. The antagonists that act as angiogenesis inhibitors are useful for the treatment of diseases where neovascularization is dominant in the pathology such as retinopathies of the eye, neovascular glaucoma, skin disorders, chronic inflammation, rheumatoid arthritis, and the like.

FGF loop structural analogues that are agonists of FGF activity can promote vascularization, cell growth, and/or cell survival, and thus have application in tissue repair such as healing of wounds, burns, bone fractures, surgical abrasions, gastrointestinal ulcers, and the like as well as tissue repair during ischemia and myocardial infarction via neovascularization of ischemic tissue.

In addition to surface loop amino acid sequence replacements, this invention further provides growth factor peptide antagonists constructed to mimic the replaced loop, *i.e.*, that bind to FGF receptors but do not stimulate proliferation or migration of fibroblasts and other cells stimulated by the corresponding factors containing the loop.

Expression of FGF receptor type is cell-specific, and the type of receptor expressed determines which FGF the cell will respond to. As mentioned above, change in receptor specificity, such as that seen for FGF-2LA, indicates that FGF-2 can be engineered to target a cell type that it normally does not interact with, such as an epithelial cell rather than an endothelial cell.

The following examples are presented to further illustrate and explain the present invention and should not be taken as limiting in any regard.

### Example 1

This example illustrates the construction of a human FGF-2 structural analogue denoted FGF-2LI (SEQ ID NO 15) having the surface loop (residues 118 to 122) of FGF-2 replaced with the corresponding amino acids derived from interleukin-1β (IL-1β).

A gene encoding human glu^{3,5}FGF-2 is first prepared as described in Seddon, A.P. *et al*., cited above, herein incorporated in its entirety by reference, and cloned into a T7 expression vector, pET-3a(M13). Briefly stated, a synthetic gene encoding the 155 amino acid form of human FGF-2 cloned into pUC 18 is purchased from British Bio-technology, Oxford, UK. The nucleotide sequence (2-49) to be changed is excised from pUC 18 with *Hind*III and *Bsp*MII and a synthetic fragment encoding the first 5 N-terminal amino acids of FGF-1 and containing an internal *Nde*1 site is cloned into pUC 18, yielding a construct encoding glutamic acid at positions 3 and 5. The cDNA encoding FGF-2 is then excised from pUC 18 with *Nde*1 and *Bam*H1 and cloned into the *Nde*1 and BamH1 restriction endonuclease sites of the expression vector pET-3a(M13), a derivative of pET-3a.

Two unique restriction endonuclease sites, *Bst*B1 and *Spl*1, are introduced into the gene in such a way as to produce no change in the encoded amino acids (i.e., silent mutations) at positions that flank the codons encoding the segment Ser117-Trp123 of FGF-2 (Figure 1).

Replacement of residues Arg118-Lys119-Tyr120-Thr121-Ser122 of FGF-2 (amino acids 118-122 of SEQ ID NO 1) with the human sequence Ala115-Gln116-Phe117-Pro118-Asn119 (amino acids 231-235 of SEQ ID NO 3) from the corresponding loop of the structural analogue IL-1β (115-119) (Figure 1) is then effected. The plasmid DNA is subjected to *Bst*B1 and *Spl*1 digestion and the larger DNA fragment, isolated using agarose gel electrophoresis. The DNA fragment is ligated using T4 DNA ligase to a doublestranded DNA obtained by annealing two synthetic oligonucleotides, 5'-CGAACGATTG GAATCTAATA ACTACAATAC GTACCGGTCT GCGCAGTTTC CTAACTGGTA TGTGGCACTT AAGC-3' (SEQ ID NO 11) and 5'-GTACGCTTAA GTGCCACATA CCAGTTAGGA AACTGCGCAG ACCGGTACGT ATTGTAGTTA TTAGATTCCA ATCGTT-3' (SEQ ID NO 12), that contain termini compatible to those generated by *Bst*B1 and *Spl*1 digestion. The ligation product is used to transform strain DH5α *E. coli* cells. The desired mutant plasmid is selected for on the basis of susceptibility to cleavage at the newly introduced *Afl*2 restriction site (underlined) and confirmed by complete sequencing of the gene. The plasmid in *E*. *coli* was deposited in the American Type Culture Collection, 12301 Parklawn Drive, Rockville, MD 20852 USA and bears A.T.C.C. accession number 69417.

### Example 2

This example illustrates the construction of a human FGF-2 structural analogue denoted FGF-2LA (SEQ ID NO 16) having the surface loop (residues 118 to 122) of FGF-2 replaced with the corresponding amino acid sequence derived from FGF-1 (Figure 1).

Replacement of the segment Arg118-Ser122 of FGF-2 with the bovine sequence Lys115-Lys116-His117-Ala118-Glu-119-Lys120-His121 (amino acids 115-121 of SEQ ID NO 2; Asn in the human sequence) corresponding to the surface loop 115-121 of FGF-1 (see Figure 1) is accomplished as described in Example 1 above using the following annealed synthetic mutagenic oligonucleotides: 5'-CGAACGATTG GAATCTAATA ACTACAATAC GTACCGGTCT AAAAAGCATG CTGAAAAACA CTGGTATGTG GCACTTAAGC-3' (SEQ ID NO 13) AND 5'-GTACGCTTAA GTGCCACATA CCAGTGTTTT TCAGCATGCT TTTTAGACCG GTACGTATTG TAGTTATTAG ATTCCAATCG TT-3' (SEQ ID NO 14). The desired mutant plasmid is selected on the basis of the susceptibility to cleavage at the newly introduced *Sph*1 restriction site (underlined) and confirmed by complete sequencing of the gene.

### Example 3

FGF-2LA and FGF-2LI mutants constructed in Examples 1 and 2 are expressed and purified in this Example.

Following sequence verification, the plasmids containing FGF-2 loop mutants described in Examples 1 and 2 above are transformed into competent *E. coli* BL21 plys S and cultured at 37°C in Luria broth containing 50 µg/ml ampicillin and 30 µg/ml chloramphenicol until an absorbance at 600 nm of 0.4 is reached. Expression of the recombinant protein is induced by the addition of 2 mM isopropylthiogalactoside for 2 hours at 37°C.

Cells from 1 liter cultures are harvested by centrifugation, resuspended in 30 ml of 50 mM Tris-HCl, pH 7.5 containing 0.1 mM EDTA and 0.6 M NaCl, and disrupted by treatment with lysozyme (10 µg/ml) for 20 min at 4°C followed by sonication (6 x 30 sec pulses). The lysates are clarified by centrifugation (10,000 x g; 20 min) and the supernatant solutions incubated with 5 ml of hydrated heparin Sepharose (Pharmacia/LKB) at 4°C for 1 hour with constant rotation. The resin is isolated by filtration on 0.8 µm filter apparatus (Nalgene), washed extensively with 10 mM Tris-HCl pH 7.4 containing 0.6 M NaCl and bound protein eluted with Tris buffer containing 3 M NaCl (25 ml). The 3 M NaCl eluent is diluted 6-fold with Tris buffer and loaded onto a TSK heparin 5PW column (0.21 x 15 cm; The Nest Group, MA) and the column developed using a linear NaCl gradient (0.6 to 2 M) in 90 min at a flow rate of 3 ml/min. residues. The proteins exhibit 36 other sites of identical and conserved residues in addition to the 7 denoted.

### Example 4

This example describes binding and cell proliferation studies using the FGF-2 mutants isolated and purified in Example 3 above.

The affinities of FGF-2LA and FGF-2LI for immobilized heparin are identical to wild type FGF-2 on elution from a TSK-heparin column at about 1.5 M NaCl.

FGF-2LI and FGF-2LA are tested for their capacity to compete for the binding of ¹²⁵I-FGF-2 (Amersham Corp.) to baby hamster kidney (BHK) cells, which express high numbers of FGF receptor. The assay employed is described by Moscatelli (*J. Cell. Physiol. 131*: 123-130 (1987)). Briefly stated, BHK cells plated on 24-well plates are incubated with 50 pM ¹²⁵I-FGF-2 with serial dilutions of unlabelled FGF-2, FGF-2LI or FGF-2LA loop mutants at room temperature for 1 hour or at 4°C for 2 hours. The cells are then incubated at 4°C for 30 minutes, washed twice with phosphate-buffered saline and treated with 20 mM HEPES (N-2-hydroxyethyl-piperazine-N'-2-ethanesulfonic acid), pH 7.5, containing 2 M NaCl to remove ¹²⁵I-FGF-2 bound to low affinity heparin sulfate binding sites. Receptor bound ¹²⁵I-FGF-2 is recovered by treatment of the cells with 0.5% Triton X-100 in 0.1 M sodium phosphate, pH 8, and counted in a γ-counter. Assays are conducted in duplicate.

The results of this binding assay, plotted in Figure 2, are found to be similar for FGF-2, FGF-2LA and FGF-2LI. Replacement of FGF-2 sequence 118-122 with the corresponding IL-1β or FGF-1 sequences has no apparent effect on the capacities of the mutant proteins to compete with ¹²⁵I-FGF-2 binding to high affinity cell membrane FGF type I receptors present on BHK cells. Since the mutants contain FGF-1 and IL-1β sequences, IL-1β and FGF-1 are tested in the BHK cell FGF-receptor binding assay. FGF-1 binds to BHK cell FGF receptors with an affinity equal to that of FGF-2, whereas no binding for hrIL-1β (Biogen) is detected.

Figure 3 shows the binding of radioidinated FGF-2 (bFGF), FGF-LA and FGF-LI to FGF-receptor type 1 presented on NIH 3T3 cells as a function of added unlabelled FGF proteins using these procedures. The data show that the competition binding curves for FGF-LA and FGF-LI are identical to that for FGF-2, and demonstrate that the loop exchanges in FGF-2 have no impact on the receptor binding properties of the proteins to cell surface FGF receptor type 1 (FGFR1).

The mitogenic activity of FGF-2, FGF-2LI and FGF-2LA are determined using bovine vascular endothelial cells derived from adult aortic arch as previously described (Gospardarowicz, D., *et al*., *Proc. Nat. Acad. Sci. 81*: 6963-6967 (1984)). Briefly, cells are seeded at an initial density of 0.8 x 10⁴ cells per 24-well plate in 0.5 ml Dulbecco's modified Eagle's medium (DMEM) containing 10% calf serum (Hyclone, Logan, UT) supplemented with penicillin (100 units/ml), streptomycin (100 ug/ml) and L-glutamine (2 mM). Two hours after plating, 20 ul aliquots of serial dilutions of FGF-2 and the mutants in DMEM are added. Inhibition of FGF-2-stimulated growth was determined at a fixed concentration of FGF-2, while that of the mutant proteins is varied. After 5 days in culture, duplicate plates are trypsinized and cell densities determined by cell counting in a Coulter counter. Determinations are conducted in duplicate.

The results of this cell proliferation assay are plotted in Figure 4. The dashed line indicates basal cell growth in the absence of added FGF-2. Closed circles indicate stimulation by FGF-2. FGF-2LI mutant is represented by open squares and FGF-2LA mutant by open triangles. FGF-2LI is about 5 to 10 times less potent than FGF-2, whereas FGF-2LA is as potent as the wild-type factor.

### Example 5

This example describes *in vitro* and *in vivo* angiogenesis studies using the FGF-2 mutants isolated and purified in Example 3 above.

Urokinase-type plasminogen activator induction by the FGF mutants is evaluated. Adult bovine aortic endothelial (ABAE) cells are seeded at 20,000 cells/well in 96-well plates and maintained in DMEM containing 10% calf serum (Hyclone, Logan, UT) supplemented with penicillin (100 units/ml), streptomycin (100 µg/ml) and L-glutamine (2 mM) and different concentrations of FGF-2 or mutants. After 24 hours, the cells are washed with phosphate-buffered saline and lysed in 60 mM Tris-HC1, pH 8.5, containing 0.05% Triton® X-100. Cell-associated urokinase-type plasminogen activator (uPA) activity is measured as described by Presta, *et al*., cited above, using the plasmin chromogenic substrate D-norleucyl-hexahydrotyrosyllysine p-nitroanilide acetate (American Diagnostics, Greenwich, CT). Cell-associated protein concentrations are determined using Coomassie blue binding to protein. The results are plotted in Figure 5. Both FGF-2LI and FGF-2LA exhibit significantly reduced capabilities to induce urokinase-type plasminogen activator.

*In vitro* angiogenesis evaluations are made by observing whether the mutants induce capillary-like structures in ABAE cells cultured on a 3-dimensional collagen gel. Three-dimensional collagen gel plates (24-well) are prepared by adding 0.5 ml chilled solution of 0.7 mg/ml rat-tail type I collagen (Becton Dickinson Labwares, Bedford, MA) containing DMEM and adjusting to neutral pH with NaHCO₃ to each well. After formation of collagen gel (about 1-2 mm thickness), ABAE cells are seeded at 50,000 cells/well. The cultures are maintained at 37°C in DMEM containing 10% calf serum (Hyclone, Logan, UT) supplemented with penicillin (100 units/ml), streptomycin (100 µg/ml) and L-glutamine (2 mM) until the cultures reach confluency, usually in 5 days by which time the cells form a monolayer on the gel. The medium is then replaced with fresh medium containing different concentrations of FGF-2 or mutants. The cultures are maintained at 37°C for 48 hours, then discontinued by fixation with cold methanol (-20°C).

The abundance of the capillary-like structures formed by ABAE cells is analyzed by using a Kontron IBAS Image Analyzer assisted with a Hamamatsu C2400 video camera and a Zeiss Axioshop microscope. The phase-contrase image of each field obtained with a video camera is converted to a binary image in which the areas occupied by a capillary-like structure is white and the rest is a black background. The extent of *in vitro* angiogenesis is then measured as a percentage of the white areas. Cell cultures of identical conditions are maintained in duplicate wells. Three to four areas of each well are examined by image analysis and the mean value and standard deviation are determined. The results from this computer-assisted quantitation method are set out in Figure 6. The induction of tube formation by mutant FGF-2LI is much less than that by wild-type FGF-2, whereas that by FGF-2LA is comparable with the wildtype.

In an *in vivo* model of angiogenesis, FGF-2LI is evaluated using the rabbit ear chamber system modified and improved by Howden, G.F., and Silver, I.A., (*Int*. *Endodontic J. 13*: 3-16 (1980)), observing the growth factor-induced neovascularization, or its inhibition, by modified growth factors or other reagents.

Rabbits are sedated using 50-70 mg/kg ketamine and 10 mg/kg xylozine administered intramuscularly. The ears are shaved with an electric hair clipper and cleaned with water, followed by Betadine®. To minimize the risk of infection, the rabbits are given 20,000 units/kg benzathine penicillin intramuscularly prior to insertion of the ear chambers. The chamber is inserted into an approximately 0.75 cm area which is not crossed by any major vessels. During the course of the experiments, observations or any minor handling of ear chambers of the animals is done by sedating the animal using the intramuscular administration of a combination of Butorphanol® and acepromazine at levels of 1 mg/kg each. FGF-2 or FGF-2LI bound to heparin-Sepharose® beads in a controlled-release alginate capsule is contained in one of the ear chambers and the other is used as a control containing only the heparin-Sepharose® alginate capsule vehicle. Thus, the effect of the growth factors and the controlled-release capsule alone can be observed simultaneously in the same animal. FGF-2 and FGF-2LI are tested at 1, 10, and 100 ng/chamber in triplicate. The animals are kept for 4 to 6 weeks and neovascularization is observed visually and recorded photographically at weekly intervals.

The criteria for evaluation of the degree of neovascularization using this procedure is set out below.

| **Criteria for Evaluation of the Degree of Neovascularization** | |
|---|---|
| *Observation* | *Score* |
| No Vascularization | 0 |
| Small Buds | 0.5 |
| Extensive Buds | 1.0 |
| Capillary Network Extending to ¼ of the Chamber | 2.0 |
| Capillary Network Extending > ¼ of the Chamber; No Anastomoses | 3.0 |
| Anastomoses from Opposite Sides of the Chamber | 4.0 |
| Extensive Anastomosing Capillary Network Filling Chamber Area | 5.0 |

The data, plotted in Figure 9, show that FGF-2LI is superior to FGF-2 in its potential to induce angiogenesis. Both FGF-2 and FGF-2LI stimulate large vessel formation, their growth across the chamber, resulting in anastomosing vessels with an active blood flow. In comparison, in control chambers that contain alginate-heparin Sepharose® beads, void of exogeneously added growth factors, only short capillaries are formed. The difference in the activity of FGF-2LI compared to FGF-2 is both quantitative and qualitative. Striking stimulatory activity of FGF-2LI compared to FGF-2 is observed over a wide range of concentrations (*i.e.*, from 0.1 - 400 ng/chamber), and the stimulation is prolific and sustained beyond the time when the stimulatory effect of FGF-2 has subsided. (See Figure 8.)

Though FGF-2 shows a dose-dependent induction of new blood vessels compared to the vehicle alone, a comparable activity for FGF-2 is observed only at concentrations of 10-100 ng/chamber; at higher dosages, a diminshed response is observed. Interestingly, at 400 ng/chamber of FGF-2, angiogenesis is completely inhibited, while FGF-2LI at the same dose greatly stimulates formation of capillaries and larger vessels. Thus, there is an observed difference in the biological activity of FGF-2LI compared to FGF-2.

Although the *in vitro* data suggest a reduced angiogenic activity for FGF-2LI, the *in vivo* studies indicate Labware Division, Becton Dickinson (Oxnard, CA). Four-well tissue culture plates are from Nunc (Rosklide, Denmark).

Soluble FGF receptor proteins are constructed by cloning of the extracellular region of murine FGF receptor 1 (FGFR-1; flg), FGF receptor 2 (FGFR-2; bek) or the KGF receptor (FGFR(IIIb); K-sam) into the alkaline phosphatase-tag expression vector, which encodes for a secreted form of placental alkaline phosphatase (AP). The FGF receptor alkaline phosphatase (FRAP) plasmids are cotransfected into NIH 3T3 cells by electroporation with a selectable neomycin resistance gene. Clonies are selected in G418 (600 µg/ml) and screened for secreted AP enzyme activity in the conditioned medium. Clones of each receptor which produced a high level of AP activity (2 to 4 A₄₀₅ units/min/ml) are then used to produce conditioned medium for binding assays.

Components of the soluble receptor binding reaction mixture include FRAP-conditioned medium (0.24 OD units/min), 2 ng/ml¹²⁵I-FGFs and 200 ng/ml heparin. The FGF:heparin:FRAP terniary complex is immunoprecipitated with 20 µl of a 1:1 slurry of anti-AP monoclonal antibodies coupled to protein A Sepharose®. All components are mixed at room temperature. The total volume is adjusted to 200 µl by addition of DMEM containing 0.1% bovine serum albumin. Binding is allowed to proceed for 1 to 2 hours at 24°C, after which time bound receptor complex or the ligand is recovered by centrifugation at 4°C (10 s at 2,000 x g). The pelleted material is washed twice with 500 µl of an ice cold buffer containing HEPES (20 mM), NaCl (150 mM), glycerol (10%) and Triton® X-100 (1%). ¹²⁵I-FGF binding is quantitated by counting of the samples in a gamma counter (LKB). Alternatively, AP enzyme activity of the FRAP protein is determined by transferring the FRAP receptor bound to heparin-Sepharose® to a flat-bottom microtiter plate in a volume of 50 µl of PBS. The reaction is initiated by addition of substrate (50 µl of 2x solution of AP assay buffer containing 2 M diethanolamine, 1 mM MgCl₂, 20 mM homoarginine and 12 mM p-nitrophenyl phosphate). The reaction is followed at room temperature at 405 nm in a kinetic microplate reader.

Receptor binding is determined by quantitating release of labelled FGF from receptors. Briefly, FGF bound to heparan sulfate low affnity sites is released from the cell surface by a 5 minute incubation with an ice cold solution containing 1.6 M Nacl, 20 mM HEPES, pH 7.4, and the amount of radioactivity release determined in a gamma-counter. FGF bound to high affinity receptors is dissociated by a 2 M NaCl (20 mM acetate buffer, pH 4.0) extraction, and the released labelled FGF is quantitated.

Chemical cross-linking experiments are carried out at room temperature in a volume of 20 µl in siliconized 0.5-ml microcentrifuge tubes. The reaction mixtures contain FGF receptor immobilized to anti-AP monoclonal antibodies coupled to protein A Sepharose®, 200 ng/ml heparin, 2 ng/ml¹²⁵I-bFGF, 20 mM phosphate buffer (pH 7.4), and 140 mM NaCl. After a 90 minute incubation, 1 ml of a solution of disuccinimidyl suberate (Pierce) dissolved in dimethyl sulfoxide is added to give a final concentration of 0.15 mM, and the mixture incubated for an additional 30 minutes. The reaction is quenched by addition of 1 ml of 200 mM ethanolamine-HCl (pH 8.0) for 30 min. The reaction mixtures are diluted 1:1 with 2x SDS-polyacrylamide gel electrophoresis loading buffer and electrophoresed on an SDS-12% polyacrylamide gel. Crosslinked FGF to the FGF receptor are detected by autoradiography on Kodak XAR film.

The position of the surface loop in FGF-2 coincides with a variable sequence region in the FGF family of proteins where various insertions occur. Since this region may be involved with determining ligand-receptor binding specificity the binding profiles of the loop mutants, LA and LI, to FGFR1 (Flg) and FGFR2 sub-type IIIb (FGF-7 or KGF receptor), which does not bind FGF-2 are determined. Figures 9A and 9B show the competition binding curves to soluble versions of FGFR1 and FGFR2(IIIb) for FGF-1, FGF-2, FGF-7 and the loop mutants following the displacement of radioiodinated FGF-2 and FGF-7 from the soluble receptor, respectively. FGF-1, FGF-2, FGF-2LA and FGF-2LI bind equally well to FGFR1, but no binding of FGF-7 is detected (Figure 9A).

The binding profiles for the binding of various FGF proteins to FGFR2(IIIb) (Figure 9B) show that FGF-7 and FGF-1 bind with the same affinity and that FGF-2 and the FGF-2L1 mutant do not bind to this receptor type; however, the binding of the LA mutant to FGFR2(IIIb) is only about 5-times less than that of FGF-7 or FGF-1. That the binding profile of FGF-2LA now mirrors that for FGF-1 and not of FGF-2, the FGF-1 loop-host protein, is consistent with the involvement of this surface loop in determining receptor-ligand specificity.

The binding specificities of radiolabelled FGF proteins and loop mutants to various soluble FGF receptors are confirmed by chemical cross-linking of the factors to the receptors in the presence and absence of an excess of the unlabelled FGF. FGF-1, FGF-2, FGF-2LA and FGF-2LI bind to FGFR1, and addition of an excess of the unlabelled FGF abolished cross-linking of ¹²⁵I-FGF. Cross-linking of ¹²⁵I-FGF-7 to FGFR1 is not detected. An identical cross-linking profile is obtained when the binding experiments are repeated using a soluble FGFR2 receptor. The cross-linking profile for FGFR2(IIIb) reveal that only FGF-1, FGF-7 and FGF-2LA bind to this receptor and that FGF-2 and the FGF-2LI mutant are excluded from binding. The cross-linking studies are consistent with the binding data presented in Figures 2, 3, and 8.

### Example 7

The FGF-7 loop sequence is introduced into FGF-2, and the properties of the new FGF-2 loop mutant are observed in this example.

A FGF-2 loop mutant denoted FGF-2LK containing the corresponding 9-residue loop sequence Ala-Lys-Trp-Thr-His-Asn-Gly-Gly-Glu from FGF-7 (residues 154-162 of SEQ ID NO 8) is constructed using the procedures outlined in Examples 1 and 2 above.

FGF-2LK shows an apparent decrease in affinity for heparin. NaCl elution from heparin-Sepharose® is about 0.7 M NaCl, compared with 1.4 M NaCl for the wild-type protein. The molarity of NaCl required to elute the LK mutant is similar to that required to elute FGF-7. The lowered affinity of the FGF-2LK mutant for heparin suggests that the loop sequence, although not directly involved in binding to heparin, is able to modify the affinity of the protein for heparin.

In receptor binding experiments like those set out in Example 6 above, the FGF-2LK protein is unable to displace or compete with the binding of ¹²⁵I-FGF-2 to FGF receptor type 1, but is able to compete with binding of ¹²⁵I-FGF-7 to FGF receptor type 2 (IIIb), whereas FGF-2 does not compete with binding. Although the potency of the FGF-2LK is about 100 times weaker than the competition observed using unlabelled FGF-7, a clear change in receptor-ligand binding profiles is observed.

The results indicate that the loop sequence from FGF-7 confers receptor-ligand specificity, and allows for the binding of the FGF-2LK mutant to a receptor subtype that binds FGF-7 but not FGF-2. Conversely, the loop sequence from FGF-7 in the FGF-2 host molecule abolishes binding of the protein to FGF receptor 1. That the affinity of the interaction is decreased by a factor of 100 suggests that other determinants in FGF-7 are involved in contributing to the binding affinity of the ligand to the receptor. The evidence also suggests that the loop sequences may modify binding to heparin.

The above description is for the purpose of teaching the person of ordinary skill in the art how to practice the present invention, and it is not intended to detail all those obvious modifications and variations of it which will become apparent to the skilled worker upon reading the description.

### SEQUENCE LISTING

(1) GENERAL INFORMATION
   (i) APPLICANTS: Andrew P. Seddon
      Luyuan Li
      Peter Böhlen
      Magdalena Eisinger
   (ii) TITLE OF INVENTION: Surface Loop Structural
      Analogues of Fibroblast Growth Factors
   (iii) NUMBER OF SEQUENCES: 16
   (iv) CORRESPONDENCE ADDRESS:
      (A) ADDRESSEE: American Cyanamid Company Patent Law Department
      (B) STREET: One Cyanamid Plaza
      (C) CITY: Fort Wayne
      (D) STATE: NJ
      (E) COUNTRY: United States
      (F) ZIP: 07470-8426
   (v) COMPUTER READABLE FORM
      (A) MEDIUM TYPE: 3.5" 1.44 mb diskette
      (B) COMPUTER: IBM PC
      (C) OPERATING SYSTEM: MS DOS
      (D) SOFTWARE: Word Processor
   (vi) PRIOR APPLICATION DATA (CIP of)
      (A) APPLICATION NUMBER: 08/126,973
      (B) FILING DATE: 09-24-93
   (viii) ATTORNEY INFORMATION
      (A) NAME: Estelle J. Tsevdos
      (B) REGISTRATION NUMBER: 31145
      (C) REFERENCE/DOCKET NUMBER: 854-008CIP (32,063)
   (ix) TELECOMMUNICATION INFORMATION
      (A) TELEPHONE NUMBER: 201-831-3242
      (B) TELEFAX NUMBER: 201-831-3305
(2) INFORMATION FOR SEQ ID NO: 1
   (i) SEQUENCE CHARACTERISTICS
      (A) LENGTH: 21
      (B) TYPE: amino acid
      (C) STRANDEDNESS: single
      (D) TOPOLOGY: linear
   (ii) MOLECULE TYPE
      (A) DESCRIPTION: peptide
   (v) FRAGMENT TYPE: internal fragment
   (ix) FEATURE
      (A) NAME: fibroblast growth factor-2 loop region
   (x) PUBLICATION INFORMATION
      (A) AUTHOR: Eriksson, A.E., *et al*.
      (B) TITLE: Three-dimensional structure of human basic fibroblast growth factor
      (C) JOURNAL: *Proc. Nat. Acad. Sci. USA*
      (D) VOLUME: 88
      (F) PAGES: 3441-3445; sequence on page 3444
      (G) DATE: April 1991
      (K) RELEVANT RESIDUES: segment corresponding to polypeptide residues 110 to 130
   (xi) SEQUENCE DESCRIPTION: SEQ ID NO 1:
(3) INFORMATION FOR SEQ ID NO: 2
   (i) SEQUENCE CHARACTERISTICS
      (A) LENGTH: 23
      (B) TYPE: amino acid
      (C) STRANDEDNESS: single
      (D) TOPOLOGY: linear
   (ii) MOLECULE TYPE
      (A) DESCRIPTION: peptide
   (v) FRAGMENT TYPE: internal fragment
   (ix) FEATURE
      (A) NAME: fibroblast growth factor-1 loop region
   (x) PUBLICATION INFORMATION
      (A) AUTHOR: Eriksson, A.E., *et al*.
      (B) TITLE: Three-dimensional structure of human basic fibroblast growth factor
      (C) JOURNAL: *Proc. Nat. Acad. Sci. USA*
      (D) VOLUME: 88
      (F) PAGES: 3441-3445; sequence on page 3444
      (G) DATE: April 1991
      (K) RELEVANT RESIDUES: segment corresponding to polypeptide residues 107 to 129
   (xi) SEQUENCE DESCRIPTION: SEQ ID NO 2:
(4) INFORMATION FOR SEQ ID NO: 3
   (i) SEQUENCE CHARACTERISTICS
      (A) LENGTH: 21
      (B) TYPE: amino acid
      (C) STRANDEDNESS: single
      (D) TOPOLOGY: linear
   (ii) MOLECULE TYPE
      (A) DESCRIPTION: peptide
   (v) FRAGMENT TYPE: internal fragment
   (ix) FEATURE
      (A) NAME: interleukin-1β loop region
   (x) PUBLICATION INFORMATION
      (A) AUTHOR: Eriksson, A.E., *et al*.
      (B) TITLE: Three-dimensional structure of human basic fibroblast growth factor
      (C) JOURNAL: *Proc. Nat. Acad. Sci. USA*
      (D) VOLUME: 88
      (F) PAGES: 3441-3445; sequence on page 3444
      (G) DATE: April 1991
      (K) RELEVANT RESIDUES: segment corresponding to polypeptide residues 223 to 243
   (xi) SEQUENCE DESCRIPTION: SEQ ID NO 3:
(5) INFORMATION FOR SEQ ID NO: 4
   (i) SEQUENCE CHARACTERISTICS
      (A) LENGTH: 37
      (B) TYPE: amino acid
      (C) STRANDEDNESS: single
      (D) TOPOLOGY: linear
   (ii) MOLECULE TYPE
      (A) DESCRIPTION: peptide
   (v) FRAGMENT TYPE: internal fragment
   (ix) FEATURE
      (A) NAME: fibroblast growth factor-3 loop region
   (x) PUBLICATION INFORMATION
      (A) AUTHOR: Miyamoto, M., *et al.*
      (B) TITLE (excerpt): Molecular cloning of a Novel Cytokine cDNA Encoding the Ninth Member of the Fibroblast Growth Factor Family
      (C) JOURNAL: *Molecular and Cellular Biology*
      (D) VOLUME: 13
      (E) NUMBER: 7
      (F) PAGES: 4251-4259; Figure 2 on page 4254
      (G) DATE: July 1993
      (K) RELEVANT RESIDUES: segment corresponding to polypeptide residues 124 to 160
   (xi) SEQUENCE DESCRIPTION: SEQ ID NO 4:
(6) INFORMATION FOR SEQ ID NO: 5
   (i) SEQUENCE CHARACTERISTICS
      (A) LENGTH: 21
      (B) TYPE: amino acid
      (C) STRANDEDNESS: single
      (D) TOPOLOGY: linear
   (ii) MOLECULE TYPE,
      (A) DESCRIPTION: peptide
   (v) FRAGMENT TYPE: internal fragment
   (ix) FEATURE
      (A) NAME: fibroblast growth factor-4 loop region
   (x) PUBLICATION INFORMATION
      (A) AUTHOR: Burgess, W.H., and Maciag, T.
      (B) TITLE: The Heparin-Binding (Fibroblast) Growth Factor Family of Proteins
      (C) JOURNAL: *Ann. Rev. Biochem.*
      (D) VOLUME: 58
      (F) PAGES: 575-606, Figure 1 on page 580
      (G) DATE: 1989
      (K) RELEVANT RESIDUES: segment corresponding to polypeptide residues 164 to 184
   (xi) SEQUENCE DESCRIPTION: SEQ ID NO 5:
(7) INFORMATION FOR SEQ ID NO: 6
   (i) SEQUENCE CHARACTERISTICS
      (A) LENGTH: 27
      (B) TYPE: amino acid
      (C) STRANDEDNESS: single
      (D) TOPOLOGY: linear
   (ii) MOLECULE TYPE
      (A) DESCRIPTION: peptide
   (v) FRAGMENT TYPE: internal fragment
   (ix) FEATURE
      (A) NAME: fibroblast growth factor-5 loop region
   (x) PUBLICATION INFORMATION
      (A) AUTHOR: Burgess, W.H., and Maciag, T.
      (B) TITLE: The Heparin-Binding (Fibroblast) Growth Factor Family of Proteins
      (C) JOURNAL: *Ann. Rev. Biochem.*
      (D) VOLUME: 58
      (F) PAGES: 575-606, Figure 1 on page 580
      (G) DATE: 1989
      (K) RELEVANT RESIDUES: segment corresponding to polypeptide residues 168 to 194
   (xi) SEQUENCE DESCRIPTION: SEQ ID NO 6:
(8) INFORMATION FOR SEQ ID NO: 7
   (i) SEQUENCE CHARACTERISTICS
      (A) LENGTH: 21 residues
      (B) TYPE: amino acid
      (C) STRANDEDNESS: single
      (D) TOPOLOGY: linear
   (ii) MOLECULE TYPE
      (A) DESCRIPTION: peptide
   (v) FRAGMENT TYPE: internal fragment
   (ix) FEATURE
      (A) NAME: fibroblast growth factor-6 loop region
   (x) PUBLICATION INFORMATION
      (A) AUTHOR: Miyamoto, M., *et al*.
      (B) TITLE (excerpt): Molecular cloning of a Novel Cytokine cDNA Encoding the Ninth Member of the Fibroblast Growth Factor Family
      (C) JOURNAL: *Molecular and Cellular Biology*
      (D) VOLUME: 13
      (E) NUMBER: 7
      (F) PAGES: 4251-4259; Figure 2 on page 4254
      (G) DATE: July 1993
      (K) RELEVANT RESIDUES: segment corresponding to polypeptide residues 166 to 186
   (xi) SEQUENCE DESCRIPTION: SEQ ID NO 7:
(9) INFORMATION FOR SEQ ID NO: 8
   (i) SEQUENCE CHARACTERISTICS
      (A) LENGTH: 25 residues
      (B) TYPE: amino acid
      (C) STRANDEDNESS: single
      (D) TOPOLOGY: linear
   (ii) MOLECULE TYPE
      (A) DESCRIPTION: peptide
   (v) FRAGMENT TYPE: internal fragment
   (ix) FEATURE
      (A) NAME: fibroblast growth factor-7 loop region
   (x) PUBLICATION INFORMATION
      (A) AUTHOR: Miyamoto, M., *et al*.
      (B) TITLE (excerpt): Molecular cloning of a Novel Cytokine cDNA Encoding the Ninth Member of the Fibroblast Growth Factor Family
      (C) JOURNAL: *Molecular and Cellular Biology*
      (D) VOLUME: 13
      (E) NUMBER: 7
      (F) PAGES: 4251-4259; Figure 2 on page 4254
      (G) DATE: July 1993
      (K) RELEVANT RESIDUES: segment corresponding to polypeptide residues 146 to 170
   (xi) SEQUENCE DESCRIPTION: SEQ ID NO 8:
(10) INFORMATION FOR SEQ ID NO: 9
   (i) SEQUENCE CHARACTERISTICS
      (A) LENGTH: 21 residues
      (B) TYPE: amino acid
      (C) STRANDEDNESS: single
      (D) TOPOLOGY: linear
   (ii) MOLECULE TYPE
      (A) DESCRIPTION: peptide
   (v) FRAGMENT TYPE: internal fragment
   (ix) FEATURE
      (A) NAME: fibroblast growth factor-8 loop region
   (x) PUBLICATION INFORMATION
      (A) AUTHOR: Tanaka, A., *et al*.
      (B) TITLE (excerpt): Cloning and Characterization of an Antrogen-Induced Growth Factor
      (C) JOURNAL: *Proc. Natl. Acad . Sci . USA*
      (D) VOLUME: 89
      (F) PAGES: 8928-8931; Figure 2 on page 8930
      (G) DATE: October 1992
      (K) RELEVANT RESIDUES: segment corresponding to polypeptide residues 136 to 156
   (xi) SEQUENCE DESCRIPTION: SEQ ID NO 9:
(11) INFORMATION FOR SEQ ID NO: 10
   (i) SEQUENCE CHARACTERISTICS
      (A) LENGTH: 27 residues
      (B) TYPE: amino acid
      (C) STRANDEDNESS: single
      (D) TOPOLOGY: linear
   (ii) MOLECULE TYPE
      (A) DESCRIPTION: peptide
   (v) FRAGMENT TYPE: internal fragment
   (ix) FEATURE
      (A) NAME: fibroblast growth factor-9 loop region
   (x) PUBLICATION INFORMATION
      (A) AUTHOR: Miyamoto, M., *et al*.
      (B) TITLE (excerpt): Molecular cloning of a Novel Cytokine cDNA Encoding the Ninth Member of the Fibroblast Growth Factor Family
      (C) JOURNAL: *Molecular and Cellular Biology*
      (D) VOLUME: 13
      (E) NUMBER: 7
      (F) PAGES: 4251-4259; Figure 2 on page 4254
      (G) DATE: July 1993
      (K) RELEVANT RESIDUES: segment corresponding to polypeptide residues 143 to 167
   (xi) SEQUENCE DESCRIPTION: SEQ ID NO 10:
(12) INFORMATION FOR SEQ ID NO: 11
   (i) SEQUENCE CHARACTERISTICS
      (A) LENGTH: 74
      (B) TYPE: nucleic acid
      (C) STRANDEDNESS: single
      (D) TOPOLOGY: linear
   (ii) MOLECULE TYPE
      (A) DESCRIPTION: oligonucleotide
   (v) FRAGMENT TYPE: synthetic DNA
   (ix) FEATURE
      (D) OTHER INFORMATION: used in preparing constructs
   (xi) SEQUENCE DESCRIPTION: SEQ ID NO 11:
   CGAACGATTG GAATCTAATA ACTACAATAC GTACCGGTCT GCGCAGTTTC   50
   CTAACTGGTA TGTGGCACTT AAGC                               74
(13) INFORMATION FOR SEQ ID NO: 12
   (i) SEQUENCE CHARACTERISTICS
      (A) LENGTH: 76
      (B) TYPE: nucleic acid
      (C) STRANDEDNESS: single
      (D) TOPOLOGY: linear
   (ii) MOLECULE TYPE
      (A) DESCRIPTION: oligonucleotide
   (v) FRAGMENT TYPE: synthetic DNA
   (ix) FEATURE
      (D) OTHER INFORMATION: used in preparing constructs
   (xi) SEQUENCE DESCRIPTION: SEQ ID NO 12:
   GTACGCTTAA GTGCCACATA CCAGTTAGGA AACTGCGCAG ACCGGTACGT   50
   ATTGTAGTTA TTAGATTCCA ATCGTT                             76
(14) INFORMATION FOR SEQ ID NO: 13
   (i) SEQUENCE CHARACTERISTICS
      (A) LENGTH: 80
      (B) TYPE: nucleic acid
      (C) STRANDEDNESS: single
      (D) TOPOLOGY: linear
   (ii) MOLECULE TYPE
      (A) DESCRIPTION: oligonucleotide
   (v) FRAGMENT TYPE: synthetic DNA
   (ix) FEATURE
      (D) OTHER INFORMATION: used in preparing constructs
   (xi) SEQUENCE DESCRIPTION: SEQ ID NO 13:
   CGAACGATTG GAATCTAATA ACTACAATAC GTACCGGTCT AAAAAGCATG 50
   CTGAAAAACA CTGGTATGTG GCACTTAAGC 80
(15) INFORMATION FOR SEQ ID NO: 14
   (i) SEQUENCE CHARACTERISTICS
      (A) LENGTH: 82
      (B) TYPE: nucleic acid
      (C) STRANDEDNESS: single
      (D) TOPOLOGY: linear
   (ii) MOLECULE TYPE
      (A) DESCRIPTION: oligonucleotide
   (v) FRAGMENT TYPE: synthetic DNA
   (ix) FEATURE
      (D) OTHER INFORMATION: used in preparing constructs
   (xi) SEQUENCE DESCRIPTION: SEQ ID NO 14:
   GTACGCTTAA GTGCCACATA CCAGTGTTTT TCAGCATGCT TTTTAGACCG 50
   GTACGTATTG TAGTTATTAG ATTCCAATCG TT 82
(16) INFORMATION FOR SEQ ID NO: 15
   (i) SEQUENCE CHARACTERISTICS
      (A) LENGTH: 471 bases encoding 155 amino acids
      (B) TYPE: nucleic acid and amino acid
      (C) STRANDEDNESS: single
      (D) TOPOLOGY: linear
   (ii) MOLECULE TYPE
      (A) DESCRIPTION: DNA encoding a protein
   (v) FRAGMENT TYPE: entire sequence
   (vi) IMMEDIATE SOURCE: constructed
   (ix) FEATURE
      (D) INFORMATION: FGF-2 having surface loop residues 118 to 122 replaced with corresponding structural elements from interleukin-1β
   (xi) SEQUENCE DESCRIPTION: SEQ ID NO 15:
(17) INFORMATION FOR SEQ ID NO: 16
   (i) SEQUENCE CHARACTERISTICS
      (A) LENGTH: 477 bases encoding 157 amino acids
      (B) TYPE: nucleic acid and amino acid
      (C) STRANDEDNESS: single
      (D) TOPOLOGY: linear
   (ii) MOLECULE TYPE
      (A) DESCRIPTION: DNA encoding a protein
   (v) FRAGMENT TYPE: entire sequence
   (vi) IMMEDIATE SOURCE: constructed
   (ix) FEATURE
      (D) INFORMATION: FGF-2 having surface loop residues 118 to 122 replaced with corresponding structural elements from bovine FGF-1
   (xi) SEQUENCE DESCRIPTION: SEQ ID NO 16:

### BIBLIOGRAPHY

Arakawa, T. and Fox G.M., Eur. Pat. Ap. Pub. No. 320,148 (1989).
Baird, A., *et al., Proc. Nat. Acad. Sci. USA 85:* 2324-2328 (1988).
Baird, A., and Böhlen, P., *Handbook of Exp. Pharmacol.* 95(1): 369-418, Springer, 1990.
Bergonzoni, L., *et al.,* Eur. Pat. Ap. Pub. No. 363,675 (1990).
Davidson, J.M., *et al*., *J. Cell Bio. 100:* 1219-1227 (1985).
Eriksson, E.A., *et al., Proc. Nat. Acad. Sci. USA 88*: 3441-3445 (1991).
Feige, J.J. *et al., Proc. Nat. Acad. Sci. USA 86*: 3174-3178 (1989).
Fiddes, J.C., *et al*., Eur. Pat. Ap. Pub. No. 298,723 (1989).
Franco, W.P., U.S. Pat. No. 4,378,347, Mar. 29, 1983.
Givol, D., and Yayon, A., *FASEB J. 6*: 3362-3369 (1992).
Gospardarowicz, D., *et al., Proc. Nat. Acad. Sci. 81*: 6963-6967 (1984).
Howden, G.F., and Silver, I.A., *Int. Endodontic J.* 13: 3-6 (1980).
Hynes, T.R., *et al*., *Nature 339:* 73-76 (1989).
Jaye, M., *et al., Biochim. Biophys. Acta 1135*: 185-199 (1992).
Johnson, D.E., and Williams, L.T., *Adv. Can. Res. 60*: 1-41 (1993).
Miyamoto, M., *et al., Mol. Cell. Biol. 13*: 4251-4259 (1993).
Moscatelli, D., *J*. *Cell. Physiol. 131:* 123-130 (1987).
Presta, M., *et al., B.B.R.C. 185:* 1098-1107 (1992).
Seddon, A.P. *et al., Annals N.Y. Acad. Sci. 638*: 98-108 (1991).
Seno, M., *et al*., *Eur. Pat.* Ap. *Pub. Nos. 281,822* (1988) and 326,907 (1989).
Seno, M., *et al., Eur. J. Biochem. 188:* 239-245 (1990).
Tanaka, A., *et al., Proc. Natl. Acad. Sci. USA 89*: 8928-8932 (1992).
Werner, S., *et al*., *Mol. Cell. Bio.* 12: 82-88 (1992).
Yayon, A., *et al., EMBO J. 11*: 1885-1890 (1992).
Zhang, J., *et al., Proc. Nat. Acad. Sci. USA 88*: 3446-3450 (1991).
Zhu, H., *et al., Science 251*: 90-93 (1991).

## Claims

1. An FGF-2 analogue in which amino acids corresponding to FGF-2 amino acids 118 to 122 of SEQ ID NO 1 have been replaced with an amino acid sequence selected from the group consisting of:
(a) FGF-1 amino acids 115-121 of SEQ ID NO 2;
(b) FGF-1 amino acids 115-121 of SEQ ID NO 2; except that His121 is replaced by Asn121;
(c) FGF-3 amino acids 132-152 of SEQ ID NO 4;
(d) FGF-4 amino acids 172-176 of SEQ ID NO 5;
(e) FGF-5 amino acids 176-186 of SEQ ID NO 6;
(f) FGF-6 amino acids 174-178 of SEQ ID NO 7;
(g) FGF-7 amino acids 154-162 of SEQ ID NO 8;
(h) FGF-8 amino acids 144-148 of SEQ ID NO 9;
(i) FGF-9 amino acids 151-161 of SEQ ID NO 10; and
(j) interleukin-1β amino acids 231-235 of SEQ ID NO 3,
wherein said analogue is capable of binding to heparin.

2. An FGF-2 analogue according to Claim 1, in which the amino acids corresponding to FGF-2 amino acids 118-122 of SEQ ID NO 1 have been replaced with an amino acid sequence selected from the group consisting of:
(a) FGF-1 amino acids 115-121 of SEQ ID NO 2;
(b) FGF-7 amino acids 154-162 of SEQ ID NO 8; and
(c) interleukin-1β amino acids 231-235 of SEQ ID NO 3.

## Patentansprüche

1. FGF-2-Analoges, in welchem die Aminosauren, die den FGF-2-Aminosäuren 118 bis 122 von SEQ ID NO 1 entsprechen, durch eine Aminosäuresequenz ersetzt wurden, die aus der Gruppe ausgewahlt ist, bestehend aus
(a) FGF-1-Aminosäuren 115 - 121 von SEQ ID NO 2,
(b) FGF-1-Aminosäuren 115 - 121 von SEQ ID NO 2
mit der Ausnahme, daß His121 durch Asn121 ersetzt ist,
(c) FGF-3-Aminosäuren 132 - 152 von SEQ ID NO 4,
(d) FGF-4-Aminosäuren 172 - 176 von SEQ ID NO 5,
(e) FGF-5-Aminosäuren 176-186 von SEQ ID NO 6,
(f) FGF-6-Aminosäuren 174 - 178 von SEQ ID NO 7,
(g) FGF-7-Aminosäuren 154 - 162 von SEQ ID NO 8,
(h) FGF-8-Aminosäuren 144 - 148 von SEQ ID NO 9,
(i) FGF-9-Aminosäuren 151 - 161 von SEQ ID NO 10 und
(j) Interleukin-1β-Aminosduren 231 - 235 von SEQ ID NO 3,
wobei das Analoge in der Lage ist, an Heparin zu binden.

2. FGF-2-Anatoges nach Anspruch 1, in dem die Aminosäuren, die den FGF-2-Aminosäuren 118 bis 122 von SEQ ID NO 1 entsprechen, durch eine Aminosäuresequenz ersetzt wurden, die aus der Gruppe ausgewählt ist, bestehend aus:
(a) FGF-1-Aminosäuren 115 - 121 von SEQ ID NO 2,
(b) FGF-7-Aminosäuren 154-162 von SEQ ID NO 8 und
(c) Interleukin-1β-Aminosäuren 231 - 235 von SEQ ID NO 3.

## Revendications

1. Analogue de FGF-2 dans lequel les aminoacides correspondants aux aminoacides 118 à 122 de la SEQ ID N° 1 de FGF-2 ont été remplacés par une séquence d'aminoacides choisie dans le groupe consistant en :
(a) les aminoacides 115 à 121 de la SEQ ID H° 2 de FGF-1 ;
(b) les aminoacides 115 à 121 de la SEQ ID N° 2 de FGF-1, sauf que His121 est remplacé par Asn121 ;
(c) les aminoacides 132 à 152 de la SEQ ID N° 4 de FGF-3 ;
(d) les aminoacides 172 à 176 de la SEQ ID N° 5 de FGF-4 ;
(e) les aminoacides 176 à 186 de la SEQ ID N° 6 de FGF-5 ;
(f) les aminoacides 174 à 178 de la SEQ ID N° 7 de FGF-6 ;
(g) les aminoacides 154 à 162 de la SEQ ID 11° 8 de FGF-7 ;
(h) les aminoacides 144 à 148 de la SEQ ID N° 9 de FGF-8 ;
(i) les aminoacides 151 à 161 de la SEQ ID N° 10 de FGF-9 ; et
(j) les aminoacides 231 à 235 de la SEQ ID N° 3 de l'interleukine-1β,
ledit analogue étant capable de se lier à l'héparine.

2. Analogue de FGF-2 suivant la revendication 1, dans lequel les aminoacides correspondant aux aminoacides 118 à 122 de la SEQ ID N° 1 de FGF-2 ont été remplacés par une séquence d'aminoacides choisie dans le groupe consistant en :
(a) les aminoacides 115 à 121 de la SEQ ID N° 2 de FGF-1 ;
(b) les aminoacides 154 à 162 de la SEQ ID N° 8 de FGF-7 ; et
(c) les aminoacides 231 à 235 de la SEQ ID N° 3 de l'interleukine-1β.
